# EUROPEAN PATENT APPLICATION

(11) **EP 1 880 668 A1**
(43) Date of publication of application: **23.01.2008**
(21) Application number: 07110668.6
(22) Date of filing: 20.06.2007
(51) Int. Cl.: A61B 5/103

(54) **Tonometer for determining the tension and consistency of body tissues**

(30) Priority: 21.07.2006 IT BO20060547
(71) Applicant: Mezzana, Paolo, 00184 Roma (IT); Pomar, Rodolfo, 40067 Pianoro (BO) (IT)
(72) Inventor: Mezzana, Paolo, 00184, ROMA (IT)
(74) Representative: Modiano, Micaela Nadia

(57) **Abstract**

A tonometer (1) for detecting the tension and consistency of the tissues of the human and/or animal body, comprising a base plate (2) on which at least one substantially flat and linear pressure detection sensor (3) is arranged. The sensor (3) is connected to a control and management unit (6) for detecting the data related to the pressure curve determined by the sensor (3).

## Description

The present invention relates to a tonometer for detecting the tension and consistency of the tissues of the human and/or animal body.

A strong increase in the consistency of certain tissues may occur as a consequence of the presence of certain disorders or after surgical procedures.

The increase in consistency (hardness and stiffness) can lead to considerable aching of the body parts and to an unnatural appearance.

In particular, after plastic surgery procedures aimed at introducing a silicone prosthesis for example in the breast, this phenomenon is particularly significant.

Since the prosthesis is made of inert material (silicone), it is not rejected by the body, but it can still be subject to the formation of layers of protective covering connective tissue, which isolates it from the tissues of the body: substantially, a sort of capsule which covers the prosthesis will form.

The consistency of the prosthesis is preset in order to match the consistency of the tissues within which it will be implanted. Upon formation of the capsule, a considerable increase in the consistency of the prosthesis-capsule complex occurs and this causes the appearance of the surgically treated breast to be substantially unnatural.

In practice, the excessive toughness entails both and an appearance which is visibly different from a "natural" breast and an uneven consistency.

Secondly, it must be noted that the presence of the capsule and the increase in consistency that it forces cause pain in the tissues in the vicinity of the prosthesis, since they are constantly subjected to unnatural pressures and mechanical actions.

In order to avoid the onset of these problems and in particular to avoid (or at least reduce) the formation of the capsule of connective tissue, the standard practice is to subject the patient who has undergone the procedure to continuous cycles of massages of the body part.

The mechanical action of the massage is intended to hinder the rigid overlap of layers of connective tissue: continuous and prolonged massage ensures that even if the connective tissue stratifies, the overlapping layers in any case maintain a consistency which is comparable to that of the other tissues that constitute the breast.

This type of massage has the severe drawback of being quite painful for the patient (since it is a very vigorous manipulation) and of having to be prolonged for a considerable period of time (even several months).

It should be noted that these massages must be performed by skilled and trained personnel and therefore also entail a considerable increase in overall costs. Correct execution of this type of massage requires identifying precisely the areas affected by a higher consistency in order to be able to target the mechanical manipulation.

The criteria for identifying the areas with highest consistency are of an exclusively subjective type and are based exclusively on tactile sensations and on the interpretation of some somatic characteristics which are visible to the eye of an expert.

The aim of the present invention is to provide a tonometer for detecting the tension and consistency of the tissues of the human and/or animal body which is capable of providing objective data for identifying areas of higher consistency.

Within this aim, an object of the present invention is to provide a tonometer for detecting the tension and consistency of the tissues of the human and/or animal body which is capable of associating numeric values, according to a preset scale, with the consistency of the various portions of tissue being analyzed, allowing to detect variations thereof during suitable treatments.

Another object of the present invention is to provide a tonometer for detecting the tension and consistency of the tissues of the human and/or animal body which has low costs, is relatively simple to provide in practice and is safe in application.

This aim and these and other objects which will become better apparent hereinafter are achieved by the present tonometer for detecting the tension and consistency of the tissues of the human and/or animal body, characterized in that it comprises a base plate on which at least one substantially flat and linear pressure detection sensor is arranged, said sensor being connected to an appropriate control and management unit for detecting the data related to the pressure curve determined by the sensor.

Further characteristics and advantages of the present invention will become better apparent from the following detailed description of a preferred but not exclusive embodiment of a tonometer for detecting the tension and consistency of the tissues of the human and/or animal body, illustrated by way of non-limiting example in the accompanying drawings, wherein:

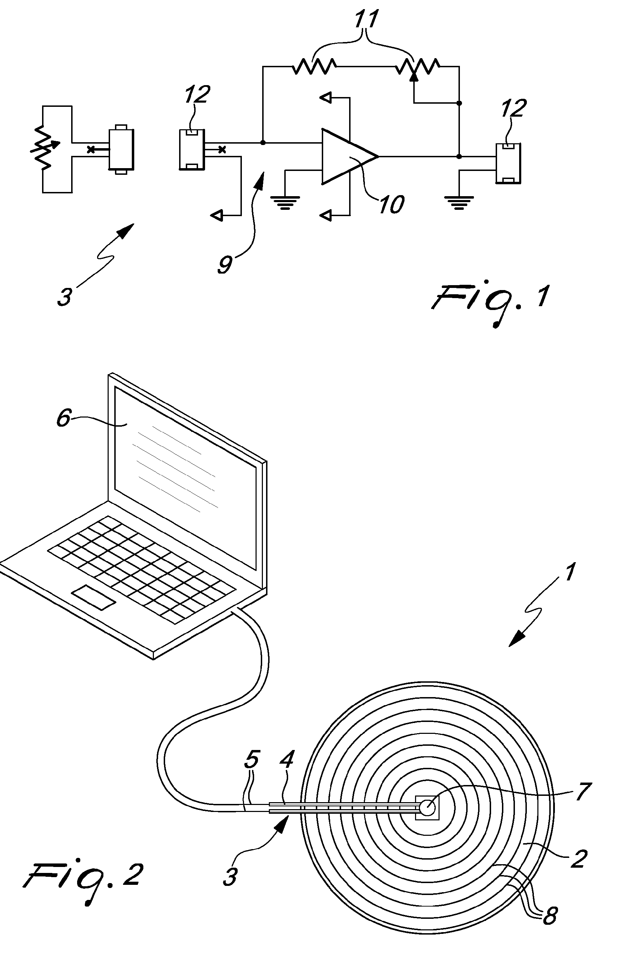

Figure 1 is a schematic view of one of the possible electronic circuits for providing a sensor of a tonometer for detecting the tension and consistency of the tissues of the human and/or animal body according to the invention;
Figure 2 is a schematic view of a first embodiment of a tonometer for detecting the tension and consistency of the tissues of the human and/or animal body with a single sensor according to the invention;
Figure 3 is a schematic view of a second embodiment of a tonometer for detecting the tension and consistency of the tissues of the human and/or animal body with a double sensor according to the invention;
Figure 4 is a schematic view of a third embodiment of a tonometer for detecting the tension and consistency of the tissues of the human and/or animal body with a triple sensor according to the invention;
Figure 5 is a schematic view of a fourth embodiment of a tonometer for detecting the tension and consistency of the tissues of the human and/or animal body with a quadruple sensor according to the invention.

With reference to the figures, the reference numeral 1 generally designates a tonometer for detecting the tension and consistency of the tissues of the human and/or animal body.

The tonometer 1 comprises a base plate 2 on which at least one pressure detecting sensor 3 is arranged.

The sensor 3 has the appearance of a ribbon 4 within which respective electrical conductors 5 are accommodated; the variations in electrical resistance which can be detected across the conductors 5 are linked to the pressure to which the various sections of the sensor 3 are subjected. The conductors 5 are connected to a suitable control and management unit 6 (which is shown in the figure as a personal computer of the standard type but might also be provided by means of a dedicated component equipped with suitable processing devices capable of processing the signal and with a screen or printing device for representing the detected data).

The data in fact transpose the pressure curve to which the sensor 3 is subjected during the tests.

According to an embodiment of particular practical and applicative interest, the sensor 3 has a first end 7 which is arranged substantially at the center of the plate 2.

It should be noted that the plate 2 is preferably substantially circular; in particular, the plate 2 must be made of transparent material (for example Plexiglas or glass) and must have a plurality of concentric circles 8 imprinted on its surface. The purpose of this embodiment is to be able to use a linear scale for correct identification of the specific position of each specific sensing. In this particular embodiment, the sensor 3 is arranged in a radial direction: the end 7 therefore coincides with the center of the circles 8 and the conductors 5 protrude outside the plate 2.

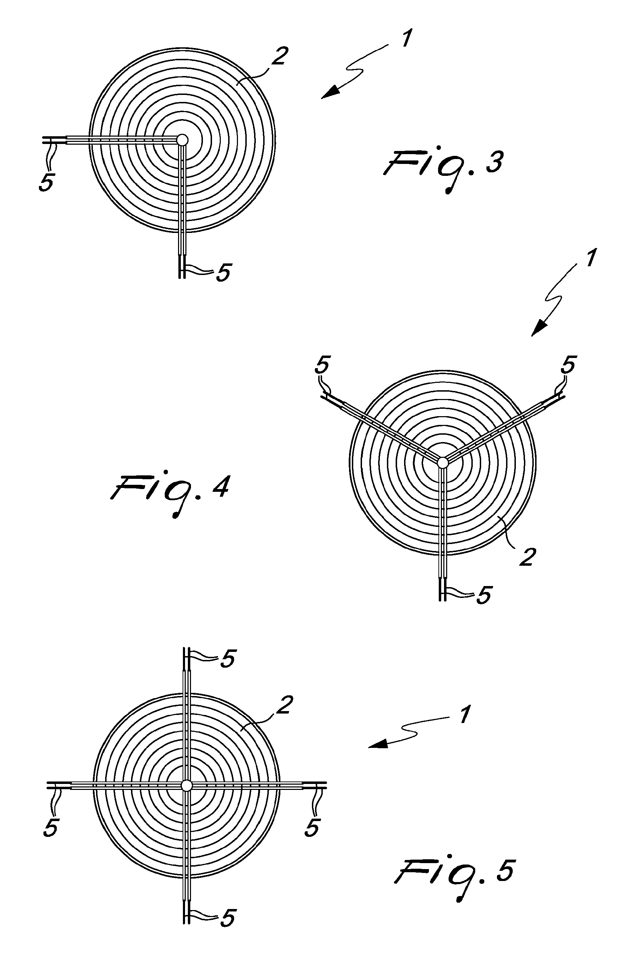

According to another embodiment, shown in Figure 3, there are two sensors 3, which are arranged radially with respect to the plate 2 and along mutually perpendicular directions.

To generalize, the sensors 3 can be a plurality arranged radially with respect to the plate 2 with a regular angular distribution: for example, it can be seen that in Figure 4 there are three sensors 3 arranged at 120° with respect to each other and in Figure 5 there are four sensors 3 arranged at 90° with respect to each other.

Of course, the use of a plurality of sensors 3 allows to map with greater precision the consistency of the tissues, allowing, for example by interpolation, to define a surface which is associated with the trend of the pressure in the area being examined (a datum which can be obtained following processing on the part of the unit 6).

According to one of the many possible embodiments, the sensor 3 comprises a respective signal conditioning and amplifying circuit 9, which is interposed between the actual sensor 3 and the management and control unit 6. The circuit 9 is constituted by an amplifier 10, respective polarization resistors 11 and suitable connectors 12.

The operation of the invention is as follows: the patient has to rest the part to be examined on the surface of the plate 2 so that the pressure of the part acts directly on the sensor 3.

In order to detect the consistency of the breast of a patient after a plastic surgery procedure, it is convenient to have the patient lie on her back (for example on a bed) and arrange the plate 2 so that it rests on the breast in a region adjacent to the areola-nipple complex.

In this manner, the sensing of the sensor 3 can be interpreted easily and allows to use the data to plan the most appropriate treatment for giving the breast a uniform consistency.

The sensor 3 that is used detects the pressure in a circular area at the center of the base 2 and not along its radius. The sensor 3 is sensitive to pressure only in its central area. The tonometer 1 operates by measuring the response of breast tissue to the pressure of a circular plate (base 2) made of Plexiglas or other transparent material. The higher the resistance offered by the breast to the weight of the base 2, the higher the pressure in the central area measuring approximately 1 square centimeter (since the entire weight of the base 2 is discharged more onto a smaller surface). The lower the consistency of the breast, the more the plate will rest on the breast and the larger the area of distribution of the weight on the plate (therefore the lower the pressure on the central point).

It has thus been shown that the invention achieves the intended aim and objects.

The invention thus conceived is susceptible of numerous modifications and variations, all of which are within the scope of the appended claims.

In particular, it should be noted that all the components might communicate with each other also by means of electromagnetic waves (without any wired connection) in order to simplify the structure of the entire invention and make it more easily usable by operators.

All the details may further be replaced with other technically equivalent ones.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the shapes and dimensions, may be any according to requirements without thereby abandoning the scope of the protection of the appended claims.

The disclosures in Italian Patent Application No. BO2006A000547 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A tonometer for detecting the tension and consistency of the tissues of the human and/or animal body, **characterized in that** it comprises a base plate (2) on which at least one substantially flat and linear pressure detection sensor (3) is arranged, said sensor (3) being connected to an appropriate control and management unit (6) for detecting the data related to the pressure curve determined by the sensor (3).

2. The tonometer according to claim 1, **characterized in that** said sensor (3) has a first end (7) which is arranged substantially at the center of said plate (2).

3. The tonometer according to claim 1, **characterized in that** said plate (2) is substantially circular.

4. The tonometer according to claims 2 and 3, **characterized in that** said sensor (3) is arranged in a radial direction.

5. The tonometer according to claim 3, **characterized in that** said plate (2) is transparent and has a plurality of concentric circles (8) imprinted on its surface.

6. The tonometer according to one or more of the preceding claims, **characterized in that** said sensors (3) are a plurality and are arranged radially with respect to the plate (2) with a regular angular distribution.

7. The tonometer according to one or more of the preceding claims, **characterized in that** said pressure detection sensor (3) comprises a respective signal conditioning and amplification circuit (9), which is interposed between the actual sensor (3) and the management and control unit (6), which is constituted by an amplifier (10), the respective polarization resistors (11) and connectors (12).

8. The tonometer according to one or more of the preceding claims, **characterized in that** said sensor (3) is of the resistive type and associates with pressure variations on its surface variations in the electrical resistance of the associated sensor portion (3).
